# EUROPEAN PATENT APPLICATION

(11) **EP 1 867 726 A1**
(43) Date of publication of application: **19.12.2007**
(21) Application number: 06729942.0
(22) Date of filing: 24.03.2006
(51) Int. Cl.: C12N 15/88

(54) **LIPOSOME CAPABLE OF EFFECTIVE DELIVERY OF GIVEN SUBSTANCE INTO NUCLEUS**

(30) Priority: 24.03.2005 JP 2005086890
(71) Applicant: National University Corporation Hokkaido University, Sapporo-shi, Hokkaido 0600808 (JP); Kyoto University, Kyoto-shi, Kyoto 606-8501 (JP); SHIONOGI & CO., LTD., Osaka-shi, Osaka 541-0045 (JP)
(72) Inventor: KOGURE, Kentaro, Kita-ku,Sapporo-shi, Sapporo-shi,Hokkaido 060-0812 (JP); NAKAMURA, Takashi, Kita-ku,Sapporo-shi, Sapporo-shi,Hokkaido 060-0812 (JP); AKITA, Hidetaka, Kita-ku,Sapporo-shi, Sapporo-shi,Hokkaido 060-0812 (JP); HARASHIMA, Hideyoshi, Sapporo-shi, Sapporo-shi,Hokkaido 060-0812 (JP); FUTAKI, Shiroh, ty, Gokasho, Uji-shi, Kyoto, 6110011 (JP)
(74) Representative: Hiebl, Inge Elisabeth
(86) International application number: PCT/JP2006/305994
(87) International publication number: WO 2006/101201

(57) **Abstract**

Disclosed is a non-viral vector capable of delivering a given substance into the nucleus of a target cell effectively even when the target cells is a undividable cell such as a dendritic cell. A bilamellar liposome having a first lipid membrane and a second lipid membrane successively from the outside, the first lipid membrane having a membrane-fusing ability and the second lipid membrane having on its surface a nuclear transport peptide.

## Description

### Technical Field

The present invention relates to non-viral vector, more particularly liposome.

### Background Art

Recently, development of vectors for certainly delivering a given substance such as drug, nucleic acid, peptide, protein, sugar, etc. into a target site is carried out. For example, viral vectors such as retrovirus, adenovirus, adeno-associated virus, and the like are developed as the vectors for transducing the given gene into a target cell. However, since the viral vectors have a number of problems such as difficulty for mass production, antigenicity, toxicity, and the like, non-viral vectors (e.g. liposome vector) having fewer such problems are receiving considerable attention, and development of non-viral vectors introduced with various functional molecules are carried out in order to increase intracellular delivery efficiency of the given substance.

For example, a liposome, to which the hydrophilic polymer (e.g. polyalkylene glycol such as polyethylene glycol, etc.) is introduced into the outer surface of the liposome membrane, is developed (refer to Patent Document 1, Patent Document 2, Patent Document 3 and Patent Document 4). Using such the liposome, directional characteristics of the liposome to tumor cells can be improved by improving retention of the liposome in blood.

A liposome, to which a substance (for example, transferrin, insulin, folic acid, hyaluronic acid, antibody or its fragments, sugar chain) capable of binding to a receptor or an antigen existing on the surface of cell membrane is introduced, has been developed (refer to Patent Document 3 and Patent Document 4). Using such the liposome, endocytic efficacy of the liposome can be improved.

Further, a liposome, in which stearylated octaarginine is introduced into the outer surface of the liposome membrane, has been developed (Non-Patent Document 1). Using such the liposome, delivery efficiency of a given substance encapsulated in the liposome into the cells can be improved.
Patent Document 1: JP-A-01-249717
Patent Document 2: JP-A-02-149512
Patent Document 3: JP-A-04-346918
Patent Document 4: JP-A-2000-10481
Non-Patent Document: Kogure, K. et al. "Journal of Controlled Release", 2004, Vol. 98, pages 317-323

### Disclosure of Invention

### Problem to be Solved by the Invention

The present inventor has found that in the delivery system of a given substance into a target cell using a conventional non-viral vector, when the target cell was a nondividing cell such as a dendritic cell, a delivery efficiency of a given substance into the nucleus (the intracellular expression efficiency of a nucleic acid when the given substance is a nucleic acid) was significantly decreased as compared with a case when the target cell is a dividing cell (refer to Reference Example 1).

Consequently, an object of the present invention is to provide a non-viral vector capable of delivering a given substance into the nucleus of a target cell effectively even when the target cell is a nondividing cell such as the dendritic cell.

### Means for Solving Problem

In order to achieve the above object, the present invention provides a bilamellar liposome described in (1) to (11) hereinbelow.
(1) A bilamellar liposome having a first lipid membrane and a second lipid membrane successively from the outside, wherein said first lipid membrane has a membrane fusion ability and said second lipid membrane has a nuclear transport peptide on the surface thereof.
(2) The bilamellar liposome according to the above-described (1), wherein said first lipid membrane contains a membrane fusion lipid as a constituent thereof.
(3) The bilamellar liposome according to the above-described (2), wherein an amount of the membrane fusion lipid contained in said first lipid membrane is 50% (in mole percentage) or more of the total amount of lipid contained in said first lipid membrane.
(4) The bilamellar liposome according to the above-described (2) or (3), wherein among the membrane fusion lipids contained in said first lipid membrane, a percentage of anionic lipid is 5 to 50% (in mole percentage).
(5) The bilamellar liposome according to any one of the above-described (1) to (4), wherein a given substance to be delivered into the nucleus of a target cell is encapsulated in the inside of said second lipid membrane.
(6) The bilamellar liposome according to the above-described (5), wherein the liposome is a vector for delivering said given substance into the nucleus of said target cell.
(7) The bilamellar liposome according to the above-described (6), wherein said target cell is a nondividing cell.
(8) The bilamellar liposome according to any one of the above-described (1) to (7), obtainable by contacting a plurality of negatively-charged SUV liposomes which contain a membrane fusion lipid as a constituent of the lipid membrane and have a nuclear transport peptide on the surface of the lipid membrane, with positively-charged particles, and by inducing membrane-fusion of the negatively-charged SUV liposomes each other which are electrostatically bound to the positively-charged particles.
(9) The bilamellar liposome according to the above-described (8), wherein said positively-charged particles are aggregates of a given substance to be delivered into the nucleus of a target cell.
(10) The bilamellar liposome according to any one of the above-described (1) to (7), obtainable by contacting a plurality of positively-charged SUV liposomes which contain a membrane fusion lipid as a constituent of the lipid membrane and have a nuclear transport peptide on the surface of the lipid membrane, with negatively-charged particles, and by inducing membrane-fusion of the positively-charged SUV liposomes each other which are electrostatically bound to the negatively-charged particles.
(11) The bilamellar liposome according to the above-described (10), wherein said negatively-charged particles are aggregates of a given substance to be delivered into the nucleus of a target cell.

### Effect of the Invention

According to the present invention, the bilamellar liposome capable of effectively delivering a given substance into the nucleus of a target cell is provided, even when the target cell is a nondividing cell such as dendritic cell.

### Brief Description of Drawings

[Fig. 1]

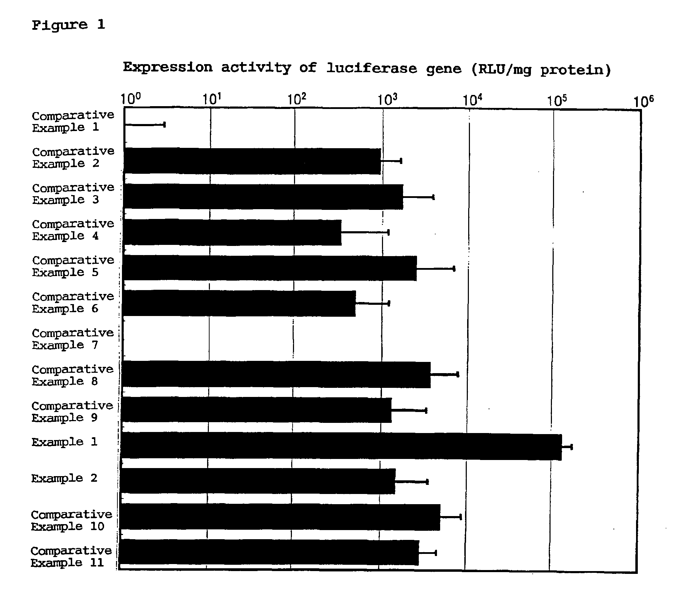

   Fig. 1 shows the results of measurement on the expression activity of luciferase gene (RLU/mg protein).
[Fig. 2]

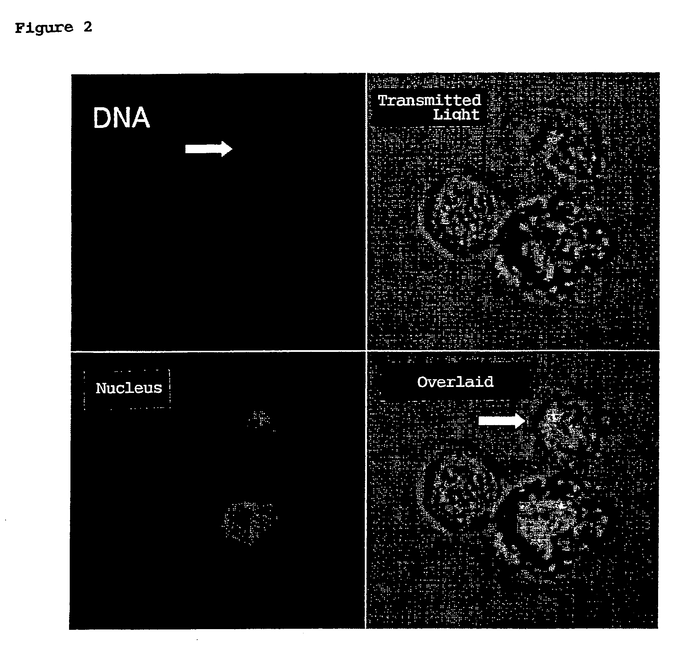

   Fig. 2 shows the results of observation by a confocal laser scanning microscope.
[Fig. 3]

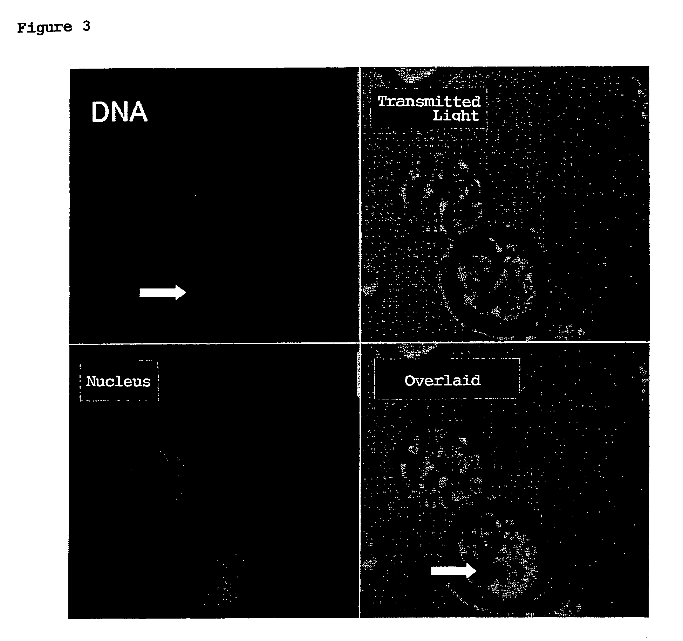

   Fig. 3 shows the results of observation by a confocal laser scanning microscope.
[Fig. 4]

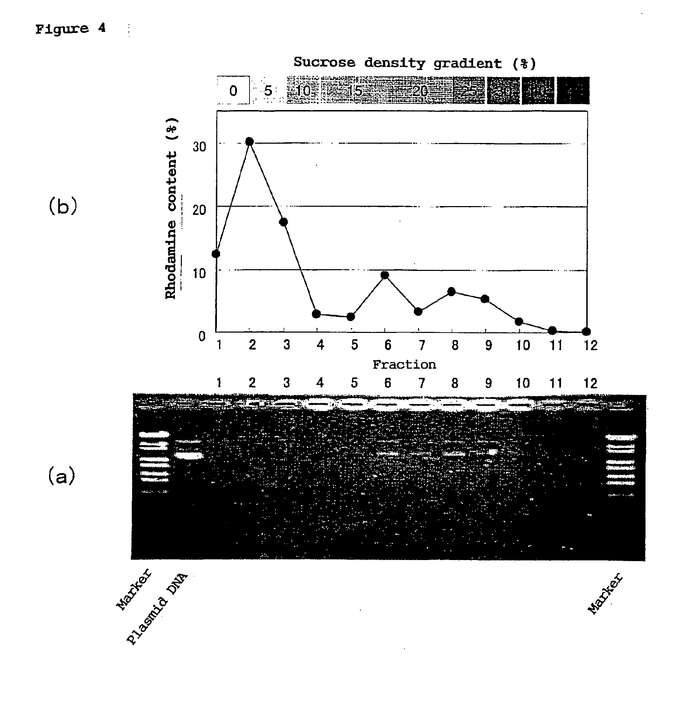

   Fig. 4 (a) shows the results of measurement on DNA content in each fraction; and Fig. 4 (b) shows the results of measurement on rhodamine content in each fraction.
[Fig. 5]

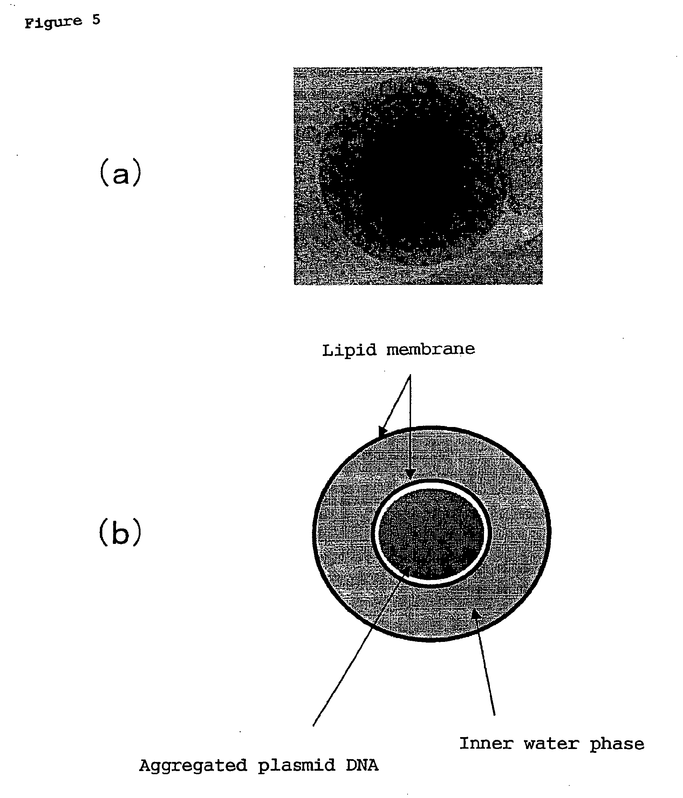

   Fig. 5 (a) shows the results of electron microscopic observation on the bilamellar liposome of the present invention, and Fig. 5(b) shows the schematic drawing of the bilamellar liposome of the present invention.
[Fig. 6]

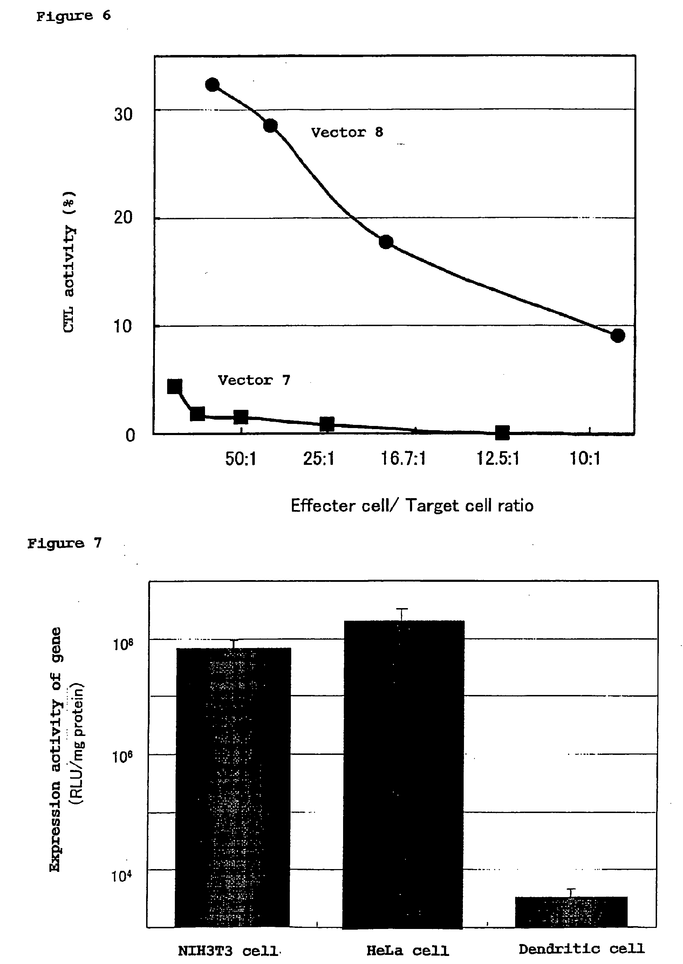

   Fig. 6 shows the results of measurement on CTL activity.
[Fig. 7]
   Fig. 7 shows the results of measurement on the expression activity of luciferase gene (RLU/mg protein).
[Fig. 8]

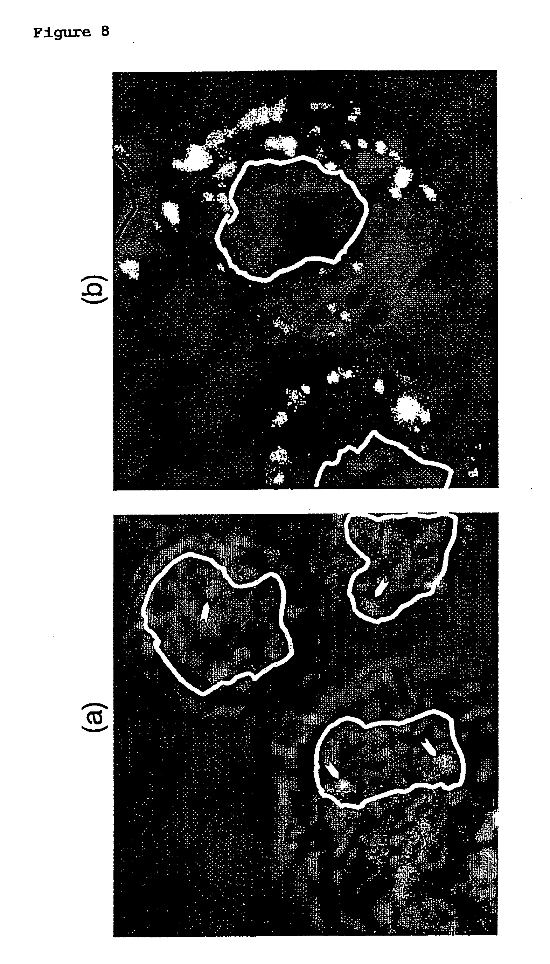

   Fig. 8 shows the results of observation by a confocal laser scanning microscope.

### Best Mode for Carrying Out the Invention

The present invention will be explained in detail hereinbelow.
The liposome of the present invention is a bilamellar liposome having a first lipid membrane and a second lipid membrane successively from the outside.
Size of the liposome of the present invention is not particularly limited, and is generally 50 to 500 nm, preferably 100 to 300 nm, and more preferably 100 to 200 nm in diameter.

In the liposome of the present invention, the first and the second lipid membranes have each a lipid bilayer structure. Constituents of the first and the second lipid membranes are not particularly limited, so long as these constituents do not inhibit formation of the lipid bilayer structure, and include, for example, lipid, membrane stabilizer, antioxidant, charged matter, membrane protein, and the like.

Lipid is an essential constituent of the lipid membrane, and amount of lipid contained in each lipid membrane is generally 30 to 100% (in mole percentage), preferably 50 to 100% (in mole percentage), and more preferably 70 to 100% (in mole percentage) in the total amount of the substance contained in each lipid membrane.

Examples of lipid include, for example, phospholipids, glycolipids, sterols and saturated or unsaturated fatty acids.

Examples of phospholipids include, for example, phosphatidyl choline (e.g. dioleoylphosphatidylcholine, dilauroylphosphatidylcholine, dimyristoylphosphatidylcholine, dipalmitoylphosphatidylcholine, distearoylphosphatidylcholine, etc.), phosphatidylglycerol (e.g. dioleoylphosphatidylglycerol, dilauroylphosphatidylglycerol, dimyristoylphosphatidylglycerol, dipalmitoylphosphatidylglycerol, distearoylphosphatidylglycerol, etc.), phosphatidylethanolamine (e.g. dioleoylphosphatidylethanolamine, dilauroylphosphatidylethanolamine, dimyristoylphosphatidylethanolamine, dipalmitoylphosphatidylethanolamine, distearoylphosphatidylethanolamine, etc.), phosphatidylserine, phosphatidylinositol, phosphatidic acid, cardiolipin, sphingomyelin, egg-yolk lecithin, soybean lecithin, and hydrogenated substance thereof.

Examples of the glycolipids include, for example, glyceroglycolipid (e.g. sulfoxyribosyl glyceride, diglycosyl diglyceride, digalactosyl diglyceride, galactosyl diglyceride and glycosyl diglyceride), sphingoglycolipid (e.g. galactosylcerebroside, lactosylcerebroside and ganglioside), and the like.

Examples of the sterol include, for example, sterol of animal origin (e.g. cholesterol, cholesteryl hemisuccinate, lanosterol, dihydrolanosterol, desmosterol and dihydrocholesterol), sterol derived from plant (phytosterol) (e.g. stigmasterol, sitosterol, campesterol andbrasicasterol), sterol derived from microorganism (e.g. thymosterol and ergosterol), and the like.

Examples of the saturated or unsaturated fatty acids include, for example, C₁₂₋₂₀ saturated or unsaturated fatty acids such as palmitic acid, oleic acid, stearic acid, arachidonic acid, myristic acid, and the like.

Lipids are classified into neutral lipid, cationic lipid (basic lipid) and anionic lipid (acidic lipid). Examples of neutral lipid include, for example, diacylphosphatidylcholine, diacylphosphatidylethanolamine, cholesterol, ceramide, sphingomyelin, cephalin, cerebroside, and the like. Examples of cationic lipid include, for example, dioctadecyldimethylammonium chloride (DODAC), N-(2,3-dioleyloxy)propyl-N,N,N-trimethylammonium (DOTMA), didodecylammonium bromide (DDAB), 1,2-dioleoyloxy-3-trimethylammonio propane (DOTAP), 3β-N-(N',N'-dimethyl-aminoethane)-carbamol cholesterol (DC-Chol), 1,2-dimyristoyloxypropyl-3-dimethylhydroxyethyl ammonium (DMRIE), 2,3-dioleyloxy-N-[2(sperminecarboxamido)ethyl]-N,N-dimethyl -1-propanaminum trifluoroacetate (DOSPA), and the like. Examples of anionic lipid are, for example, cardiolipin, diacylphosphatidylserine, diacylphosphatidic acid, N-succinyl-phosphatidylethanolamine (N-succinylPE), phosphatidic acid, phosphatidylinositol, phosphatidylglycerol, phosphatidylethylene glycol, cholesteryl hemisuccinate, and the like.

Membrane stabilizer is any constituent of the lipid membrane, which can be contained for physically or chemically stabilizing the lipid membrane or for adjusting fluidity of the lipid membrane. Amount of the lipid stabilizer contained in each lipid membrane is generally 10 to 50% (in mole percentage), preferably 20 to 50% (mole percentage), more preferably 30 to 50% (in mole percentage) to the total amount of the substance contained in each lipid membrane.

Examples of the membrane stabilizer include, for example, sterol, glycerol or fatty acid ester thereof. Examples of sterol include the specific examples similar to those described above, and examples of fatty acid ester of glycerol include, for example, triolein, trioctanoin, and the like.

Antioxidant is any constituent of the lipid membrane, which can be contained for preventing oxidation of the lipid membrane. Amount of the antioxidant contained in each lipid membrane is generally 5 to 30% (in mole percentage), preferably 10 to 30% (in mole percentage), and more preferably 20 to 30% (in mole percentage) to the total amount of the substance contained in each lipid membrane.

Examples of the antioxidant include, for example, tocophenol, propyl gallate, ascorbyl palmitate, butylated hydroxytoluene, and the like.

Charged matter is any constituent of the lipid membrane, which can be contained for providing a positive electric charge or a negative electric charge to the lipid membrane. Amount of the charged matter contained in each lipid membrane is generally 5 to 30% (in mole percentage), preferably 10 to 20% (in mole percentage), and more preferably 10 to 15% (in mole percentage) to the total amount of the substance contained in each lipid membrane.

Examples of the charged matter for providing positive electric charge include, for example, saturated or unsaturated aliphatic amine such as stearylamine, oleylamine, and the like, and saturated or unsaturated cationic synthetic lipid such as dioleoyltrimethylammonium propane, and the like. Examples of the charged matter for providing negative electric charge include, for example, dicetylphosphate, cholesteryl hemisuccinate, phosphatidylserine, phosphatidylinositol, phosphatidic acid, and the like.

Membrane protein is any constituent of the lipid membrane, which can be contained for maintaining the structure of the lipid membrane and providing functionality to the lipid membrane. Amount of the membrane protein contained in each lipid membrane is generally 0.1 to 2% (in mole percentage), preferably 0.5 to 2% (in mole percentage), and more preferably 1 to 2% (in mole percentage) to the total amount of the substance contained in each lipid membrane.

Examples of the membrane protein include, for example, peripheral membrane protein, integral membrane protein, and the like.

In the liposome of the present invention, the first lipid membrane has a membrane fusion ability.

The first lipid membrane, which is a membrane fusible lipid membrane, is not particularly limited so long as it has a lipid bilayer structure. Examples of the first lipid membrane, which is a membrane fusible lipid membrane, include, for example, biomembrane such as cell membrane, endosome, and the like.

Method for imparting membrane fusion ability to the first lipid membrane is not particularly limited.
For example, the membrane fusion ability can be imparted to the first lipid membrane by adding the membrane-fusible lipid as a constituent in the first lipid membrane. Examples of the membrane-fusible lipid include, for example, neutral lipid such as dioleoylphosphatidylethanolamine, and the like, anionic lipid (acidic lipid) such as cholesteryl hemisuccinate, cardiolipin, and the like, and cationic lipid (basic lipid) such as dialkyl ammonium bromide, and the like.

Optimum pH at which the neutral lipid can exhibit the membrane fusion ability is generally 6.0 to 7.5, and preferably 6.0 to 7.0, and optimum pH at which the anionic lipid can exhibit the membrane fusion ability is generally 5.0 to 6.5, and preferably 5.5 to 6.0, further, optimum pH at which the cationic lipid can exhibit the membrane fusion ability is generally 7.5 to 8.5, and preferably 8.0 to 8.5. The anionic lipid, which can exhibit the membrane fusion ability under the condition of an acidic environment, and the cationic lipid, which can exhibit the membrane fusion ability under the condition of an alkaline environment, are referred to as pH-sensitive lipid.

Amount of the membrane-fusible lipid contained in the first lipid membrane is not particularly limited so long as the membrane fusion ability can be imparted to the first lipid membrane. Amount of the membrane-fusible lipid contained in the first lipid membrane is generally 50 to 100% (in mole percentage), preferably 60 to 100% (in mole percentage), and more preferably 70 to 100% (in mole percentage) of amount of the total lipid contained in the first lipid membrane. When an amount of the membrane-fusible lipid contained in the first lipid membrane is within the above range, since the first lipid membrane can be effectively membrane-fused with the biomembrane such as cell membrane, endosome membrane, and the like, the liposome of the present invention can be internalized effectively into the cells.

Among the membrane-fusible lipids contained in the first lipid membrane, ratio of the anionic lipid is preferably 5 to 50% (in mole percentage), and more preferably 10 to 30% (in mole percentage). When ratio of the anionic lipid is within the above range and the liposome of the present invention is placed under acidic environment, the first lipid membrane can be effectively membrane-fused with the biomembrane such as cell membrane, endosome membrane, and the like. The membrane-fusible lipid except for the anionic lipid contained in the first lipid membrane may be either one or may be both of the neutral lipid and the cationic lipid.

Further, the membrane fusion ability can be imparted to the first lipid membrane by modifying the surface of the first lipid membrane with a membrane fusible molecule. Examples of the membrane fusible molecule for modifying the surface of the first lipid membrane include, for example, succinylated polyglycidol, membrane-fusible peptide (e.g. H2 peptide, GM 225.1), and the like.

A lipid or a lipid derivative having blood retention function, susceptibility function for temperature changes, etc. may be contained in the first lipid membrane as a constituent thereof. Due to the constituent, one or two or more of the above functions can be imparted to the liposome of the present invention. As a result of imparting the blood retention function to the liposome of the present invention, the retentivity of the liposome of the present invention in blood can be improved, and a trapping ratio by the reticuloendothelial tissue in liver, spleen, etc. can be reduced. Further, as a result of imparting the susceptibility function for temperature changes to the liposome of the present invention, releasing ability of the given substance encapsulated in the liposome of the present invention can be increased.

Examples of the blood retentive lipid or lipid derivative, which can be imparted the blood retention function, include, for example, glycophorin, ganglioside GM1, phosphatidylinositol, ganglioside GM3, glucuronic acid derivative, glutamic acid derivative, polyglycerol phospholipid derivative, and polyethylene glycol derivative such as N-{carbonyl-methoxypolyethylene glycol-2000}-1,2-dipalmitoyl-sn-glycero-3-phosphoethanolami ne, N-{carbonyl-methoxypolyethylene glycol-5000}-1,2-dipalmitoyl-sn-glycero-3-phosphoethanolami ne, N-{carbonyl-methoxypolyethylene glycol-750}-1,2-distearoyl-sn-glycero-3-phosphoethanolamin, N-{carbonyl-methoxypolyethylene glycol-2000}-1,2-distearoyl-sn-glycero-3-phosphoethanolamin, N-{carbonyl-methoxypolyethylene glycol-5000}-1,2-distearoyl-sn-glycero-3-phosphoethanolamin, and the like. Examples of the temperature change susceptible lipid or lipid derivative which can be imparted the susceptibility function for temperature changes are, for example, dipalmitoyl phosphatidyl choline, and the like.

In the liposome of the present invention, the second lipid membrane has a nuclear transport peptide on the surface thereof.

The nuclear transport peptide is not particularly limited so long as it has nuclear transport ability from cytoplasm to an intranuclear location and is, for example, a peptide having a nuclear transport signal. The nuclear transport signal is a specific amino acid sequence possessed by the protein or the peptide transported selectively to the nucleus. Examples thereof include a nuclear transport signal derived from simian virus 40 (SV40-NLS) (SEQ ID NO:2), a core peptide mu derived from adeno virus (SEQ ID NO:3), a nuclear transport signal derived from simian virus 40 large T antigen (SEQ ID NO:4), a repeated sequence thereof (for example, SEQ ID NO:1 (a repetitive sequence of the nuclear transport signal derived from large T antigen of simian virus 40)), and the like. The nuclear transport peptide may be a natural type peptide having the nuclear transport signal or may be a variant peptide fused with the nuclear transport signal. Total number of amino acid residues constructing the nuclear transport peptide are not particularly limited, and are generally 5 to 30 amino acid residues, preferably 5 to 25 amino acid residues, and more preferably 5 to 20 amino acid residues.

Amount of the nuclear transport peptide existing on the surface of the second lipid membrane is not particularly limited, and is generally 1 to 5% (in mole percentage), preferably 1 to 3% (in mole percentage), and more preferably 1 to 2% (in mole percentage) to the total amount of the substances contained in the second lipid membrane. When an amount of the nuclear transport peptide existing on the surface of the second lipid membrane is within the range hereinabove, the given substance encapsulated in the liposome of the present invention can be delivered effectively into the nucleus.

The nuclear transport peptide may exist on the inner surface of the second lipid membrane so long as it exists on the outer surface of the second lipid membrane. It should be noted that, among the both surfaces of second lipid membrane, the surface on the first lipid membrane side is the outer surface, and the surface on the side opposite thereto is the inner surface. Further, the nuclear transport peptide may exist on the outer surface and/or on the inner surface of the first lipid membrane.

An illustrative embodiment of the nuclear transport peptide on the surface of the second lipid membrane is such one that, for example, the nuclear transport peptide is modified with a functional group or a compound (e.g. hydrophobic group, hydrophobic compound, and the like), by which the nuclear transport peptide can be a constituent of the lipid membrane, and the functional group or the compound is inserted in the second lipid membrane, and the nuclear transport peptide is exposed from the second lipid membrane. In such the illustrative embodiment, the entire nuclear transport peptide may be exposed from the second lipid membrane, or a part of the nuclear transport peptide may be exposed from the second lipid membrane so long as the nuclear transport peptide can exhibit the transporting ability from the cytoplasm to the nucleus.

The functional group or the compound modifying the nuclear transport peptide, by which the nuclear transport peptide can be a constituent of the lipid membrane, is not particularly limited, and includes, for example, saturated or unsaturated fatty acid group such as stearyl group, and the like; cholesterol group or derivatives thereof; phospholipids, glycolipids or sterol; long chain aliphatic alcohol (e.g. phosphatidylethanolamine, cholesterol, and the like); polyoxypropylene alkyl; glycerin fatty acid ester, and the like. Among them, C₁₀₋₂₀ fatty acid group (e.g. palmitoyl group, oleyl group, stearyl group, arachidoyl group, and the like) is particularly preferable.

Method for preparing the liposome of the present invention is not particularly limited. For example, the liposome of the present invention can be prepared by contacting a plurality of negatively-charged SUV liposomes, which contain the membrane fusion lipids as a constitute of the lipid membrane and have the nuclear transport peptide on the surface of the lipid membrane, with the positively-charged particles, and causing the membrane fusion of the negatively-charged SUV liposomes themselves, which are electrostatically bound to the positively-charged particles. Further, the liposome of the present invention can also be prepared by contacting a plurality of positively-charged SUV liposomes, which contain the membrane fusion lipids as a constitute of the lipid membrane and have the nuclear transport peptide on the surface of the lipid membrane, with the negatively-charged particles, and causing the membrane fusion of the positively-charged SUV liposomes themselves, which are electrostatically bound to the negatively-charged particles.

Zeta potential of the positively-charged particles is not particularly limited so long as the potential is maintained to be positive, and is generally 10 to 60 mV, preferably 20 to 50 mV, and more preferably 20 to 40 mV. Zeta potential of the negatively-charged particles is not particularly limited so long as the potential is maintained to be negative, and is generally -10 to -60 mV, preferably -20 to -50 mV, and more preferably -20 to -40 mV. When zeta potential of the positively-charged particles or the negatively-charged particles is within the above range, the liposome of the present invention can be produced effectively. In addition, measurement condition of zeta potential is not particularly limited, and temperature condition is generally at 25°C.

Particle size of the positively-charged particles and the negatively-charged particles is not particularly limited, and lower limit of the particle size is preferably 50 nm, and more preferably 70 nm, and upper limit of the particle size is preferably 500 nm, and more preferably 200 nm. When particle size of the positively-charged particles or the negatively-charged particles is within the above range, the liposome of the present invention can be effectively produced.

The positively-charged particles may contain a neutral substance and/or an anionic substance in addition to a cationic substance so long as the particles are positively charged as a whole. The negatively-charged particles may contain a neutral substance and/or a cationic substance in addition to an anionic substance so long as the particles are negatively charged as a whole.

As the positively-charged particles or the negatively-charged particles, for example, aggregates of a given substance to be delivered into the nucleus of the target cells can be used. When the aggregates of a given substance are used as the positively-charged particles or the negatively-charged particles, the bilamellar liposome encapsulating the given substance in the inner side of the second lipid membrane can be produced. The aggregates of a given substance may be constituted of the given substance alone or may contain substances other than the given substance (e.g. carrier maintaining the given substance).

When a given substance is positively charged, the aggregates of the given substance can be prepared, for example, by electrostatically binding the given substance to the anionic substance to form a complex. When a given substance is negatively charged, the aggregates of the given substance can be prepared, for example, by electrostatically binding the given substance to the cationic substance to form a complex. When a given substance is charged neither positively nor negatively, the aggregates of the given substance can be prepared by binding the given substance to the cationic substance in proper manner (for example, physical adsorption, hydrophobic bonding, chemical bonding, and the like) to form complex. When performing the complex formation, the aggregates of the positively- or negatively-charged given substance as a whole can be prepared by regulating a mixing ratio of the given substance and the cationic substance or the anionic substance.

Examples of a given substance are not particularly limited, and include, for example, nucleic acid, peptide, protein, drug, sugar, complex thereof, and the like. In addition, "nucleic acid" includes, in addition to DNA or RNA, analogues or derivatives thereof (e.g. peptide nucleic acid (PNA), phosphorothioate DNA, and the like). Further, the nucleic acid may be single-stranded or double-stranded, and linear or cyclic form.

When a given substance is nucleic acid, the aggregate of nucleic acid can be prepared by electrostatically binding the nucleic acid to the cationic substance to form a complex. When forming a complex, the aggregates of the positively- or negatively-charged nucleic acid as a whole can be prepared by regulating a mixing ratio of the nucleic acid and the cationic substance.

The cationic substance used in the preparation of the aggregates of a given substance is not particularly limited so long as the substance has cationic group in the molecule. Examples of the cationic substance include, for example, cationic lipid (e.g. Lipofectamine (Invitrogen Inc.)); polymer having cationic groups; homopolymer or copolymer of basic amino acid such as polylysine, polyarginine, copolymer of lysine and arginine, or derivatives thereof (e.g. stearylated derivatives); polycationic polymer such as polyethyleneimine, poly(allylamine), poly(diallyldimethylammonium chloride), glucosamine, and the like; protamine or derivatives thereof (e.g. protamine sulfate), and the like. Among them, protamine or derivatives thereof is preferred. Since the aggregate of a given substance prepared by using protamine or a derivative thereof has very flexible structure, the aggregate of a given substance can be passed effectively through the pore of nuclear membrane. Number of cationic group possessed by the cationic substance is not particularly limited, and is preferably two or more. The cationic group is not particularly limited so long as it can be positively charged, and includes, for example, amino group; monoalkylamino group such as methylamino group, ethylamino group, and the like; dialkylamino group such as dimethylamino group, diethylamino group, and the like; imino group; guanidine group, and the like can be mentioned.

The anionic substance used in the preparation of the aggregates of a given substance is not particularly limited so long as the substance has anionic group in the molecule. As the anionic substance, for example, anionic lipid; polymer having anionic groups; homopolymer or copolymer of acidic amino acid such as polyaspartic acid or derivatives thereof; polyanionic polymer, etc. such as xanthan gum, carboxyvinyl polymer, carboxymethyl cellulose polystyrene sulfonate, polysaccharide, carrageenan, and the like can be used. Number of anionic group possessed by the anionic substance is not particularly limited, and is preferably two or more. The anionic group is not particularly limited so long as it can be negatively charged, and includes, for example, functional group having a terminal carboxyl group (e.g. succinic acid residue, malonic acid residue, and the like), phosphate group, sulfate group, and the like.

SUV (small unilamellar vesicle) type of liposome is a unilamellar liposome having a particle size (diameter) of 100 nm or less. The particle size (diameter) of SUV liposome is not particularly limited so long as it is 100 nm or less, and is generally 30 to 100 nm, preferably 30 to 80 nm, and more preferably 30 to 60 nm.

Since multilamellar vesicle (MLV) and unilamellar liposome other than SUV (e.g. LUV (large unilamellar vesicle), GUV (giant unilamellar vesicle), and the like) have a particle size of 100 nm or more (generally, a lipid membrane having a particle size of 100 nm or more is thought to be a plane membrane), curvature and surface energy of the lipid membrane are so small and the aggregation of liposomes themselves is difficult to occur. Contrary to that, since SUV liposome has a particle size less than 100 nm, curvature and surface energy of the lipid membrane are so large and the aggregation of liposomes themselves is easy to occur. Consequently, SUV liposome themselves containing a membrane fusion lipid as a constituent of the lipid membrane can be effectively membrane-fused.

The SUV liposome having the membrane fusion lipid as a constituent of the lipid membrane and the nuclear transport peptide on the surface of the lipid membrane can be produced, for example, as follows. Namely, after constituents of the lipid membrane (including the membrane fusion lipid) and nuclear transport peptide modified with a functional group or a compound, by which the nuclear transport peptide can be a constituent of the lipid membrane, are dissolved in an organic solvent, the organic solvent is removed by evaporation to obtain the lipid membrane. In this case, examples of the organic solvent include, for example, hydrocarbons such as pentane, hexane, heptane, cyclohexane, and the like; halogenated hydrocarbons such as methylene chloride, chloroform, and the like; aromatic hydrocarbons such as benzene, toluene, and the like; lower alcohols such as methanol, ethanol, and the like; esters such as methyl acetate, ethyl acetate, and the like; ketones such as acetone; and the like, and these can be used alone or in combination of two or more types. Subsequently, the lipid membrane is hydrated and stirred or subjected to ultrasonication using an ultrasonic device to prepare multilamellar liposome. Subsequently, the multilamellar liposome is treated with ultrasonication using a probe ultrasonic device to prepare the small unilamellar liposome, i.e. SUV type liposome. Thus, the SUV liposome, in which a functional group or a compound which can be a constituent of the lipid membrane is inserted into the lipid membrane, and that the nuclear transport peptide is exposed from the surface of the lipid membrane, can be produced.

Further, after preparing SUV liposome without having the nuclear transport peptide on the surface of the lipid membrane, into its external fluid, the nuclear transport peptide modified by a functional group or a compound which can be a constituent of the lipid membrane is added, thereby a functional group or a compound which can be a constituent of the lipid membrane is inserted into the lipid membrane, and then the SUV liposome in which the nuclear transport peptide is exposed from the surface of the lipid membrane can be produced.

Type and amount of the constituent of the lipid membrane of the positively-charged SUV liposome are regulated so that the SUV liposome is positively charged as a whole, and type and amount of the constituent of the lipid membrane of the negatively-charged SUV liposome are regulated so that the SUV liposome is negatively charged as a whole. Examples of the constituent of the lipid membrane imparting the positive charge include, for example, cationic lipid, cationic membrane stabilizer, and the like, and examples of the constituent of the lipid membrane imparting the negative charge include, for example, anionic lipid, anionic membrane stabilizer, and the like. The positively-charged SUV liposome may, so long as it is charged positively as a whole, contain a constituent of the lipid membrane imparting the negative charge and/or a neutral constituent of the lipid membrane in addition to a constituent of the lipid membrane imparting the positive charge. The negatively-charged SUV liposome may, so long as it is charged negatively as a whole, contain a constituent of the lipid membrane imparting the positive charge and/or a neutral constituent of the lipid membrane in addition to a constituent of the lipid membrane imparting the negative charge. When the positively-charged SUV liposome contains the cationic lipid as a constituent of the lipid membrane imparting the positive charge, amount of the cationic lipid is generally 5 to 30% (in mole percentage), preferably 10 to 25% (in mole percentage), and more preferably 10 to 20% (in mole percentage) of the total amount of the lipid. When the negatively-charged SUV liposome contains the anionic lipid as a constituent of the lipid membrane imparting the negative charge, amount of the anionic lipid is generally 5 to 30% (in mole percentage), preferably 10 to 25% (in mole percentage), and more preferably 10 to 20% (in mole percentage) of the total amount of the lipid.

Type and amount of the membrane fusion lipid contained in the lipid membrane of the SUV liposome are similar to the type and amount of the membrane fusion lipid contained in the second lipid membrane of the liposome of the present invention. Type and amount of the nuclear transport signal existing on the surface of the lipid membrane of the SUV liposome are similar to the type and amount of the nuclear transport signal existing on the surface of the second lipid membrane of the liposome of the present invention.

Zeta potential of the positively-charged SUV liposome is not particularly limited so long as the potential is maintained to be positive, and is generally 10 to 60 mV, preferably 20 to 50 mV, and more preferably 20 to 30 mV. Zeta potential of the negatively-charged SUV liposome is not particularly limited so long as the potential is maintained to be negative, and is generally -10 to -60 mV, preferably -20 to -50 mV, and more preferably -20 to -30 mV. When zeta potential of the positively-charged SUV liposome or the negatively-charged SUV liposome is within the above range, the liposome of the present invention can be produced effectively. In addition, measurement condition of zeta potential is not particularly limited and temperature condition is generally at 25°C.

Conditions for contacting the negatively-charged SUV liposome or the positively-charged SUV liposome with the positively-charged particles or the negatively-charged particles, respectively, are not particularly limited, and are generally 10 to 40°C, and preferably 20 to 30°C in temperature, generally at pH 6.5 to 8.0, and preferably at pH 7.0 to 7.5, and generally 1 to 20 minutes, and preferably 5 to 10 minutes in time. Solvents used in contacting are not particularly limited, and, for example, HEPES buffer, physiological saline, sucrose solution, and the like can be used. Amount of the negatively-charged SUV liposome or the positively-charged SUV liposome dispersed into the solvent is generally in excess to the positively-charged particles or the negatively-charged particles, and is, for example, 2 to 4 times of the theoretically required minimum amount of the negatively-charged SUV liposome or the positively-charged SUV liposome for encapsulating the positively-charged particles or the negatively-charged particles.

When the positively-charged particles are in contact with the negatively-charged SUV liposomes, the negatively-charged SUV liposomes are bound electrostatically to the positively-charged particles, and the negatively-charged SUV liposomes bound electrostatically to the positively-charged particles are membrane-fused with each other, then the positively-charged particles are coated with two lipid membranes. Also, when the negatively-charged particles are in contact with the positively-charged SUV liposomes, the positively-charged SUV liposomes are bound electrostatically to the negatively-charged particles, and the positively-charged SUV liposomes bound electrostatically to the negatively-charged particles are membrane-fused with each other, then the negatively-charged particles are coated with two lipid membranes. As a result, two lipid membranes coating the positively-charged particles or the negatively-charged particles are constructed on the outer side of the positively-charged particles or the negatively-charged particles to produce the liposome of the present invention. In the thus produced liposome of the present invention, the first and the second lipid membranes contain the membrane fusion lipids as a constituent thereof and have the nuclear transport signal on the surface thereof. When aggregates of a given substance are used as the positively-charged particles or the negatively-charged particles, the bilamellar liposome encapsulating the aggregates of a given substance in the inside of the second lipid membrane is produced.

When a ratio of the neutral lipid is 50% or more (in mole percentage), preferably 70% or more (in mole percentage) among the membrane fusion lipids contained in the lipid membrane of the negatively-charged SUV liposome, after the positively-charged particles are in contact with the negatively-charged SUV liposome, the membrane fusion of the negatively-charged SUV liposome bound electrostatically to the positively-charged particles with each other can be induced rapidly and effectively. The case of the positively-charged SUV liposome is same as in the case of the negatively-charged SUV liposome.

The bilamellar liposome encapsulating a given substance in the inside of the second lipid membrane can be used as a vector for delivering the given substance into the nucleus of the target cell.
The target cell may be any of a diving cell or a nondividing cell, and the unilamellar liposome encapsulating a given substance in the inside of the second lipid membrane is particularly useful when the target cell is the nondividing cell. Examples of the diving cell include, for example, NIH3T3 cell, HeLa cell, COS7 cell, and the like, and examples of the nondividing cell include, for example, the dendritic cell, the brain neuron, and the like.

The liposome of the present invention can be transported into the target cell through endocytosis. Although the liposome of the present invention transported in the target cell through the endocytosis is internalized in the endosome, it can be released from the endosome as a result of membrane fusion of the endosome membrane with the first lipid membrane. When a ratio of anionic lipid is 5 to 50% (in mole percentage) in the membrane fusion lipid contained in the first lipid membrane, the first lipid membrane and the endosome membrane can be effectively membrane-fused by changing pH in the endosome to acidic (pH 5.6 to 6.5). In the liposome released from the endosome, although the first lipid membrane has disappeared, the second lipid membrane is maintained. The nuclear transport peptide which is possessed on the surface of the second lipid membrane by the liposome released from the endosome, forms a nuclear pore targeting complex together with importin-α and -β, and is delivered into the nucleus. In this occasion, the second lipid membrane is destroyed and the given substance encapsulated in the inside of the second lipid membrane is released and delivered into the nucleus. When the aggregate of the given substance is encapsulated in the inside of the second lipid membrane, a delivery efficiency of the given substance into the nucleus is improved. When the aggregate of the given substance prepared by using protamine or its derivative is encapsulated, since the aggregate of the given substance can pass through the nuclear membrane pore effectively, the delivery efficiency of the given substance into the nucleus is particularly improved.

Further, the liposome of the present invention can be transported into the target cell mediated by the membrane-fusion of the first lipid membrane and the cell membrane. When a ratio of anionic lipid is 5 to 50% (in mole percentage) in the membrane fusion lipid contained in the first lipid membrane, the first lipid membrane and the cell membrane can be effectively membrane-fused by contacting the liposome of the present invention with the target cell under the acidic condition. In the liposome transported into the target cell, although the first lipid membrane has disappeared, the second lipid membrane is maintained. The nuclear transport peptide which is possessed on the surface of the second lipid membrane by the liposome transported into the target cell, forms the nuclear pore targeting complex together with importin-α and -β, and is delivered into the nucleus. As a result, the second lipid membrane is destroyed and the given substance encapsulated in the inside of the second lipid membrane is released and delivered into the nucleus. When the aggregate of the given substance is encapsulated in the inside of the second lipid membrane, a delivery efficiency of the given substance into the nucleus is improved. When the aggregate of the given substance prepared by using protamine or its derivative is encapsulated, since the aggregate of the given substance can pass through the nuclear membrane pore effectively, the delivery efficiency of the given substance into the nucleus is particularly improved.

The biological species, from which the cell for delivering the given substance is derived, is not particularly limited, and includes any animals, plants, microorganisms, and the like, but is preferably animals, and more preferably mammals. Examples of mammals include human, monkeys, bovine, sheep, goat, equine, swine, rabbit, canine, feline, rats, mice, guinea pig, and the like. Further, types of the cells delivering the given substance are not particularly limited, and include, for example, somatic cells, germ cells, stem cells, or cultured cells thereof, and the like.

The liposome of the present invention can be formulated in accordance with conventional methods and used as the pharmaceutical composition. The liposome of the present invention can be used, for example, in the state of dispersion. As the medium for the dispersion, for example, buffers such as physiological saline, phosphate buffer, citrate buffer, acetic acid buffer, and the like can be used. The dispersion may be used by adding, for example, additives such as sugars, polyalcohol, water-soluble polymer, non-ionic surface active agent, antioxidant, pH regulator, hydration promoter, and the like.

The liposome of the present invention can also be used in any of in vivo and in vitro. When the liposome is used in vivo, examples of the route of administration include parenteral administration such as intravenous, intraperitoneal, subcutaneous, transnasal administrations, and the like. Applied dose and number of administration can be adjusted depending on type and amount of the given substance encapsulated in the liposome.

### EXAMPLES

### Examples 1 to 2, Comparative Examples 1 to 11

### 1. Preparation of various vectors

Various vectors containing a plasmid DNA were prepared by using luciferase gene expression plasmid pcDNA3.1(+)luc as the plasmid DNA. In this connection, the plasmid pcDNA3.1(+)luc was the plasmid DNA with full length of about 7 kbp containing CMV promoter and luciferase gene linked in the downstream of the promoter, and was produced by inserting the luciferase gene, which was prepared by cutting pGL3 plasmid (Promega Corp.) with restriction enzyme, into the plasmid pcDNA3.1(+) (Invitrogen Corp.)

### (1) Vector 1 (conventional unilamellar liposome)

To a protamine sulfate solution (0.15 mL, concentration of protamine sulfate: 0.1 mg/mL, solvent: 10 mM HEPES buffer (pH 7.4)), a plasmid DNA solution (0.1 mL, concentration of plasmid DNA: 0.1 mg/mL, solvent: 10 mM HEPES buffer (pH 7.4)) was added dropwise under vortexing condition, and protamine sulfate and the plasmid DNA were allowed to be electrostatically interacted each other to condense the plasmid DNA. Particle size of the aggregated plasmid DNA was about 106 nm and zeta potential was about 39 mV. It should be noted that the hydrodynamic diameter was measured by the quasi-elastic light scattering method, and the zeta potential was analyzed by the electrophoretic light-scattering spectroscopy (ELS-8000) (hereinafter, the same as above).

After a lipid mixture (dioleoylphosphatidylethanolamine (DOPE) : cholesteryl hemisuccinate (CHEMS) = 9 : 2 (in molar ratio)) was dissolved in chloroform, chloroform was removed by evaporation to obtain a lipid membrane. To the lipid membrane (137.5 nmol), the condensed plasmid DNA suspension (0.25 mL, concentration of the condensed plasmid DNA: 0.04 mg/mL, solvent: 10 mM HEPES buffer (pH 7.4)) was added for hydration, then the condensed plasmid was bound electrostatically to the surface of the lipid membrane. Subsequently, the mixture was treated with mild ultrasonication and the condensed plasmid DNA was coated with the lipid membrane to prepare the unilamellar membrane liposome encapsulating the plasmid DNA in the inside thereof (hereinafter, designated as "vector 1").

### (2) Vector 2 (conventional carrier peptide)

To a stearylated octaarginine solution (0.09 mL, concentration of stearylated octaarginine: 0.1 mg/mL, solvent: 10 mM HEPES buffer (pH 7.4)), a plasmid DNA solution (0.18 mL, concentration of plasmid DNA: 0.1 mg/mL, solvent: 10 mM HEPES buffer (pH 7.4)) was added dropwise under vortex condition, and stearylated octaarginine and the plasmid DNA were allowed to be electrostatically interacted each other to prepare the aggregated plasmid DNA (hereinafter, designated as "vector 2").

### (3) Vector 3 (conventional unilamellar liposome)

To a stearylated octaarginine solution (0.09 mL, concentration of stearylated octaarginine: 0.1 mg/mL, solvent: 10 mM HEPES buffer (pH 7.4)), a plasmid DNA solution (0.18 mL, concentration of plasmid DNA: 0.1 mg/mL, solvent: 10 mM HEPES buffer (pH 7.4)) was added dropwise under vortex condition, and stearylated octaarginine and the plasmid DNA were allowed to be electrostatically interacted each other to condensate the plasmid DNA. The thus prepared condensed plasmid DNA was coated with the lipid membrane in the same manner as in the above (1) to prepare unilamellar liposome encapsulating the plasmid DNA in the inside thereof (hereinafter, designated as "vector 3").

### (4) Vector 4 (conventional unilamellar liposome)

After a lipid mixture (DOPE : cholesterol = 7 : 3 (in molar ratio)) was dissolved in chloroform, chloroform was removed by evaporation to obtain a lipid membrane. Using the thus obtained lipid membrane, the condensed plasmid DNA was coated with the lipid membrane in the same manner as in the above (1) to prepare the liposome encapsulating the plasmid DNA. To the suspension of the liposome encapsulating the plasmid DNA, a solution of stearylated nuclear transport peptide (the nuclear transport peptide having stearylated N-terminal (SEQ ID NO:1)) (0.05 mL, concentration of stearylated nuclear transport peptide: 1 mg/mL, solvent: 10 mM HEPES buffer (pH 7.4)) was added, and incubated at room temperature for predetermined time to distribute stearylated octaarginine on the surface of the liposome (amount of distribution of stearylated octaarginine: 2 mole % of the amount of lipid). In such way, the unilamellar liposome having octaarginine on its surface and encapsulating the plasmid DNA in the inside thereof (hereinafter, designated as "vector 4") was prepared.

### (5) Vector 5 (conventional unilamellar liposome)

To a solution of the stearylated nuclear transport peptide (the nuclear transport peptide having stearylated N-terminal (SEQ ID NO:1)) (0.09 mL, concentration of stearylated nuclear transport peptide: 0.1 mg/mL, solvent: 10 mM HEPES buffer (pH 7.4)), a solution of plasmid DNA (0.18 mL, concentration of plasmid DNA: 0.1 mg/mL, solvent: 10 mM HEPES buffer (pH 7.4)) was added dropwise under vortex condition, and the stearylated nuclear transport peptide and the plasmid DNA were allowed to be electrostatically interacted each other to prepare the aggregated plasmid DNA. The thus prepared aggregated plasmid DNA was coated with the lipid membrane by the same manner as in the above (1) to prepare the unilamellar liposome encapsulating the plasmid DNA in the inside thereof (hereinafter, designated as "vector 5").

### (6) Vector 6 (conventional unilamellar liposome)

After a lipid mixture (DOTAP : DOPE = 1 : 1 (in molar ratio)) was dissolved in chloroform, chloroform was removed by evaporation to obtain a lipid membrane. After hydration of the lipid membrane with 10 mM HEPES buffer (pH 7.4), the liposome was prepared by ultrasonication using the ultrasonic device. By adding a plasmid DNA solution (0.125 mL, concentration of plasmid DNA: 0.1 mg/mL, solvent: 10 mM HEPES buffer (pH 7.4)) to the liposome suspension (amount of lipid 137.5 nmol), a unilamellar liposome which formed a complex with the plasmid DNA (hereinafter, designated as "vector 6") was prepared.

### (7) Vector 7 (Conventional unilamellar liposome)

After a lipid mixture (DOPE : CHEMS = 9 : 2 (in molar ratio)) was dissolved in chloroform, chloroform was removed by evaporation to obtain a lipid membrane. Using the thus obtained lipid membrane, the condensed plasmid DNA was coated with the lipid membrane by the same manner as in the above (1) to prepare the liposome encapsulating the plasmid DNA. To the suspension of the liposome encapsulating the plasmid DNA, a solution of stearylated octaarginine (0.12 mL, concentration of stearylated octaarginine: 1 mg/mL, solvent: 10 mM HEPES buffer (pH 7.4)) was added, and incubated at room temperature for predetermined time to distribute stearylated octaarginine on the surface of the liposome (amount of distribution of stearylated octaarginine: 5 molar % of the amount of lipid). In this way, the unilamellar liposome having octaarginine on its surface and encapsulating the plasmid DNA in the inside thereof (hereinafter, designated as "vector 7") was prepared.

### (8) Liposome 8 (bilamellar liposome of the present invention)

After the stearylated nuclear transport peptide (the nuclear transport peptide having stearylated N-terminal (SEQ ID NO:1)) was added to a lipid mixture (DOPE : CHEMS = 9 : 2 (in molar ratio)) (amount of addition: 2 mole % of the amount of lipid) and the mixture was dissolved in chloroform (0.25 mL), the solution was picked up into a glass test tube. After removing the solvent by blowing nitrogen gas, the remained material was dried for 1 hour in a desiccator. The obtained lipid membrane (275 nmol) was hydrated by using HEPES buffer (0.25 mL) which had been warmed up to 25°C in advance and subjected to ultrasonication by using an ultrasonic device to delaminate the lipid membrane. By further treating with ultrasonication for 10 minutes using a probe type ultrasonic device, SUV liposome containing a membrane fusion lipid in the lipid membrane and having a nuclear transport peptide on its surface of the lipid membrane was prepared. Particle size of the thus prepared SUV liposome was about 58 nm, and zeta potential was about -2 mV. (This measured value has a high probability of incorrectness. When the surface of the lipid membrane has no nuclear transport peptide, zeta potential of SUV liposome is about -40 mV, therefore when a nuclear transport peptide is present on the surface of the lipid membrane, zeta potential of SUV liposome may be about -10 - -20 mV.)

To a solution of the aggregated plasmid DNA prepared by the same manner as in the above (1) (0.25 mL, concentration of aggregated plasmid DNA: 0.04 mg/mL, solvent: 10 mM HEPES buffer (pH 7.4)), a solution of SUV liposome (0.25 mL, concentration of SUV liposome: 1.1 µmol/mL, solvent: 10 mM HEPES buffer (pH 7.4)) was added and allowed to stand at room temperature (about 25°C) for 10 minutes. During this process, a plurality of SUV liposomes were electrostatically bound to the aggregated plasmid DNA and SUV liposomes were membrane-fused with each other, then the aggregated plasmid was coated with the lipid membrane, thereby preparing the liposome encapsulating the aggregated plasmid in the inside thereof. Particle size of the thus prepared liposome encapsulating the plasmid DNA (hereinafter, designated as "liposome 8") is about 160 nm and zeta potential was about 4 mV.

### (9) Liposome 9

The liposome encapsulating the plasmid DNA (hereinafter, designated as "liposome 9*"*) was prepared by the same manner as in the above (8) except that no stearylated nuclear transport peptide was added to the limpid mixture.

### 2. Transfection to the dendritic cell

Bone marrow precursor cells of C57BL/6 mouse, male, were cultured for 6 to 7 days in the presence of GM-CSF (granulocyte macrophage colony-stimulating factor) to induce immature dendritic cells. The immature dendritic cells were seeded in the 48 well plate with a density of 2 × 10⁵ cells/well, added a vector equivalent to 0. 8 µg DNA, and cultured in the serum-free medium (RPMI 1640 medium) (0.2 mL) or in a weakly-acidic buffer (10 mM HEPES/2.5% glucose (pH 6.0) (0.2 mL) at 37°C for 1 hour, thereafter added a medium (0. 5 mL) containing serum, then further cultured at 37°C for 23 hours. After culturing, cells were collected and lysed, then a reagent for assaying luciferase activity (luciferase assay system, Promega Inc.) was added to the cell lysate, and the luciferase activity was measured by using the luminometer (Luminescencer PSN, ATTO). Amount of protein in the cell lysate was measured by using BCA protein quantification kit (PIERCE, Rockford (IL)).

In Example 1, the vector 8 was used as a vector, and the weakly-acidic buffer was used in the culture. In Example 2, the vector 8 was used as a vector, and the serum-free medium was used in the culture. In addition, the vector 8 was delivered into the cell by membrane-fusion cholesteryl hemisuccinate with the cell membrane in Example 1, and the vector 8 was delivered into the cell by endocytosis in Example 2.

In Comparative example 1, the vector 1 was used as a vector, and the weakly-acidic buffer was used in the culture. In Comparative Example 2, the vector 1 was used as a vector, and the weakly-acidic buffer added with lipopolysaccharide (LPS) was used in the culture. In Comparative example 3, the vector 2 was used as a vector, and the serum-free medium was used in the culture. In Comparative Example 4, the vector 3 was used as a vector, and the serum-free medium was used in the culture. In Comparative Example 5, the vector 3 was used as a vector, and the weakly-acidic buffer was used in the culture. In Comparative Example 6, the vector 4 was used as a vector, and the serum-free medium was used in the culture. In Comparative Example 7, the vector 5 was used as a vector, and the weakly-acidic buffer was used in the culture. In Comparative Example 8, the vector 6 was used as a vector, and the serum-free medium was used in the culture. In Comparative example 9, the vector 7 was used as a vector, and the serum-free medium was used in the culture. In Comparative example 10, the vector 9 was used as a vector, and the weakly-acidic buffer was used in the culture. In Comparative example 11, the vector 9 was used as a vector, and the serum-free medium was used in the culture.

Results of measuring activities of luciferase gene expression (RLU/mg protein) in Examples 1 and 2 and Comparative Examples 1 to 10 are shown in Fig. 1. As shown in Fig. 1, the gene expression activity in Example 1 (i.e. a delivering efficiency of gene into the nucleus) was 100 times higher or more than the gene expression activities in Comparative Examples 1 to 10. In addition, although the gene expression activity in Example 2 was lower than the gene expression activity in Example 1, this is thought to be caused by low endocytic efficiency of the liposome 8. Consequently, if the endocytic efficiency is improved by modifying the surface of the lipid membrane of the liposome 8 with an appropriate molecule, the gene expression activity (i.e. a delivering efficiency of gene into the nucleus) may be improved.

### Example 3, Comparative Example 12

A vector was prepared by using a rhodamine (red) labeled plasmid DNA, and was transfected into the immature dendritic cells having the nucleus stained with CYTO 24 (green), and after 3 hours, the cells were observed by a confocal laser scanning microscope.
In Example 3, the vector 8 was transfected into the immature dendritic cells in the same manner as in Example 1. In Comparative Example 12, the vector 7 was transfected into the immature dendritic cells in the same manner as in Comparative Example 9.

Results of observation by the confocal laser scanning microscope in Example 3 were shown in Fig. 2 and Fig. 3. As shown in Fig. 2, cells, in which the plasmid DNA (red) exists as particles in the nucleus (green), were confirmed. Further, as shown in Fig. 3, cells, in which the plasmid DNA (red) is dispersed in the nucleus (green), were also confirmed. On the other hand, in Comparative Example 12, although cells, in which the plasmid DNA (red) exists in the cytoplasm, were confirmed, cells, in which the plasmid DNA (red) exists in the nucleus (green), were not confirmed.

### Example 4

The liposome 8 was prepared in the same manner as in the above (8) except that NBD (4-nitrobenzo-2-oxa-1,3-diazole) labeled plasmid DNA was used; the aggregated plasmid DNA was prepared by using poly-L-lysine; and when SUV liposome was prepared, rhodamine as an aqueous phase marker was added to HEPES buffer used for hydration of the lipid membrane (i.e. SUV liposome holding the inner water phase containing rhodamine was prepared).

The liposome suspension was layered over the discontinuous sucrose density gradient (0 to 60%), and treated by an ultracentrifuge at 160,000 × g, at 20°C for 2 hours. Fractions of 1 mL each were recovered from the upper part. DNA content in each fraction was assayed by using agarose gel electrophoresis, and fluorescence intensity of rhodamine in each fraction was measured to calculate the content of rhodamine (%).

Results of assaying DNA content of each fraction are shown in Fig. 4 (a), and results of measuring rhodamine content of each fraction are shown in Fig. 4 (b).
In fractions 6 to 9 with high DNA content, which are thought to be the fractions containing liposome 8, rhodamine was found to coexist with DNA together. From this fact, it was indicated that SUV liposomes electrostatically bound to the aggregated plasmid DNA were fused each other while the inner water phase containing rhodamine was held, to prepare the liposome 8, and a space holding the inner water phase containing rhodamine existed in the inside of the liposome 8.

Further, in fractions 6 to 9, fluorescent energy transmission between NBD and rhodamine was not observed. From this fact, it was indicated that NBD labeled plasmid DNA and the inner water phase containing rhodamine were not in contact with each other in the liposome 8, more specifically, the space holding the inner water phase containing rhodamine and the space holding the aggregated plasmid DNA were separated by the lipid membrane in the liposome 8. Consequently, the liposome 8 is thought not to be unilamellar liposome. If the liposome 8 is the unilamellar liposome, the inner water phase containing rhodamine and the aggregated plasmid DNA are held in the same space, and the fluorescence energy transfer between NBD and rhodamine should be observed.

Further, as a result of observing the liposome 8 by an electron microscope, two lipid membranes surrounding the aggregated plasmid DNA existing as a core was observed as shown in Fig. 5(a).

From the above results, it was shown that the aggregated plasmid DNA was coated with two lipid membranes by the membrane-fusion of SUV liposomes, which were electrostatically bound to the aggregated plasmid DNA, and the inner water phase containing rhodamine was held in the space between two lipid membranes newly formed in the outside of the aggregated plasmid DNA (refer to Fig. 5 (b)).

### Example 5

The vector 8 was transfected into the immature dendritic cells in the same manner as in Example 1. After 6 hours of the transfection, the cells were treated with lipopolysaccharide (LPS) for 1 hour, and were administered subcutaneously into the same line of mice (dose: 1 × 10⁵ cells). Administration was repeated to the same mice in every week, and after five weeks, cytotoxic (CTL) activity was assayed.
Further, the vector 7 was transfected into the immature dendritic cells in the same manner as in Comparative Example 9 to assay CTL activity in the same manner as above.

CTL activity was assayed by the method described below. After 5 weeks of immunization, the spleen was isolated from C57BL/6 mice, male, and the spleen cells were collected. The collected cells were seeded in 12 well plate with concentration of the cells being adjusted to 5 × 10⁵ cells/mL, then the antigen protein (luciferase) (50 µg/mL) was added thereto, and the cells were incubated at 37°C for 4 days to activate T-cell in the spleen cells (effecter cells). To the P815 cells derived from the mouse expressing the antigen protein (luciferase) in advance, radioactive isotope ⁵¹Cr was taken in to prepare the target cells, and the effecter cells in various ratio were added to the target cells, then antigen specific ⁵¹Cr leakage from the target cells by an action of the effecter cells was counted to evaluate the specific cytotoxic activity (CTL activity).

Results of assay for CTL activity are shown in Fig. 6. As shown in Fig. 6, in the case of transducing the vector 8, a significantly higher CTL activity was induced as compared with the case of transducing the vector 7.

### Example 6

The liposome 8 was prepared in the same manner as in the above (8) except that NBD (4-nitrobenzo-2-oxa-1,3-diazole) (green) labeled DOPE was added to the lipid mixture (amount of addition: 0.45 mole % of the total lipid), and that rhodamine (red) labeled plasmid DNA was used.

After incubating the immature dendritic cells in the presence of energy metabolism inhibitors (antimycin A, NaN₃ and NaF were added to give final concentration of 1 µg/mL, 0.1% and 10 mM, respectively) for 30 minutes, the vector 8 was transfected to the immature dendritic cells (the nucleus was stained with Hoechst (blue)), which were treated with the energy metabolism inhibitors (refer to Example 1), and observation by a confocal laser scanning microscope was carried out. The vector 8 was also transfected to the immature dendritic cells without treating with the energy metabolism inhibitors in the same manner as above, and observation by a confocal laser scanning microscope was carried out.

Results of the observation by a confocal laser scanning microscope are shown in Fig. 8. In this connection, in Fig. 8, (a) shows the results of the immature dendritic cells without treating with the energy metabolism inhibitors, and (b) shows the results of the immature dendritic cells treated with the energy metabolism inhibitors. Further, in Fig. 8, the white outline shows an outer edge of the nucleus, and the white arrow shows the plasmid DNA (red).

As shown in Fig. 8, the plasmid DNA (red) was observed in the nucleus (blue) in the immature dendritic cells without treating with the energy metabolism inhibitors. However, no lipid (green) was observed in the nucleus (blue). On the other hand, neither the plasmid DNA (red) nor the lipid (green) was observed in the nucleus (blue) in the case of the immature dendritic cells treated with the energy metabolic inhibitors, and the plasmid DNA (red) and the lipid (green) were observed in the cytoplasm. From these results, it was suggested that the delivery of gene into the nucleus by the vector 8 was an energy-dependent phenomenon, more specifically, an active phenomenon through the nuclear membrane pore complex existing in the nuclear membrane.

### Reference Example 1

The vector 7 was transfected into the immature dendritic cell, NIH3T3 cell or HeLa cell in the same manner as in Comparative Example 9, and the luciferase gene expression activity (RLU/mg protein) was assayed. Results are shown in Fig. 7. As shown in Fig. 7, the gene expression activity in the nondividing cell such as the dendritic cell (i.e. gene delivery efficiency in the nucleus) was significantly lower than the gene expression activity in the diving cell such as NIH3T3 cell or HeLa cell (i.e. gene delivery efficiency in the nucleus).

## Claims

1. A bilamellar liposome having a first lipid membrane and a second lipid membrane successively from the outside, wherein said first lipid membrane has a membrane fusion ability and said second lipid membrane has a nuclear transport peptide on the surface thereof.

2. The bilamellar liposome according to claim 1, wherein said first lipid membrane contains a membrane fusion lipid as a constituent thereof.

3. The bilamellar liposome according to claim 2, wherein an amount of the membrane fusion lipid contained in said first lipid membrane is 50% (in mole percentage) or more of the total amount of lipid contained in said first lipid membrane.

4. The bilamellar liposome according to claim 2 or 3, wherein among the membrane fusion lipids contained in said first lipid membrane, a percentage of anionic lipid is 5 to 50% (in mole percentage).

5. The bilamellar liposome according to any one of claims 1 to 4, wherein a given substance to be delivered into the nucleus of a target cell is encapsulated in the inside of said second lipid membrane.

6. The bilamellar liposome according to claim 5, wherein the liposome is a vector for delivering said given substance into the nucleus of said target cell.

7. The bilamellar liposome according to claim 6, wherein said target cell is a nondividing cell.

8. The bilamellar liposome according to any one of claims 1 to 7, obtainable by contacting a plurality of negatively-charged SUV liposomes, which contain a membrane fusion lipid as a constituent of the lipid membrane and have a nuclear transport peptide on the surface of the lipid membrane, with positively-charged particles, and inducing membrane-fusion of the negatively-charged SUV liposomes with each other, which are electrostatically bound to the positively-charged particles.

9. The bilamellar liposome according to claim 8, wherein said positively-charged particles are aggregates of a given substance to be delivered into the nucleus of a target cell.

10. The bilamellar liposome according to any one of claims 1 to 7, obtainable by contacting a plurality of positively-charged SUV liposomes, which contain a membrane fusion lipid as a constitutent of the lipid membrane and have a nuclear transport peptide on the surface of the lipid membrane, with negatively-charged particles, and inducing membrane-fusion of the positively-charged SUV liposomes with each other, which are electrostatically bound to the negatively-charged particles.

11. The bilamellar liposome according to claim 10, wherein said negatively-charged particles are aggregates of a given substance to be delivered into the nucleus of a target cell.


SEQUENCE LISTING

<110> Hokkaido University
<110> Kyoto University

<120> A liposome to delivery efficiently a target substance to a nucleus

<160> 4

<210> 1
<211> 15
<212> PRT
<213> Simian virus 40

<400> 1
Gly Gly Pro Lys Lys Lys Arg Lys Val Pro Lys Lys Lys Arg Lys
1               5                   10                  15

<210> 2
<211> 11
<212> PRT
<213> Simian virus 40

<400> 2
Pro Lys Lys Lys Arg Lys Val Glu Asp Pro Tyr
1               5                   10

<210> 3
<211> 19
<212> PRT
<213> Adenovirus

<400> 3
Met Arg Arg Ala His His Arg Arg Arg Arg Ala Ser His Arg Arg Met
1               5                   10                  15

Arg Gly Gly

<210> 4
<211> 7
<212> PRT
<213> Simian virus 40

<400> 4
Pro Lys Lys Lys Arg Lys Val
1               5

<110>  National University Corporation Hokkaido University
       Kyoto University
       Shionogi & Co., Ltd.

<120>  Liposome capable of effective delivery of given substance into
       nucleus

<130>  18039EP

<140>  EP 06 729 942.0
<141>  2006-03-24

<150>  JP2005-086890
<151>  2005-03-24

<160>  4

<170>  PatentIn version 3.3

<210>  1
<211>  15
<212>  PRT
<213>  Simian virus 40

<400>  1

Gly Gly Pro Lys Lys Lys Arg Lys Val Pro Lys Lys Lys Arg Lys
1               5                   10                  15


<210>  2
<211>  11
<212>  PRT
<213>  Simian virus 40

<400>  2

Pro Lys Lys Lys Arg Lys Val Glu Asp Pro Tyr
1               5                   10


<210>  3
<211>  19
<212>  PRT
<213>  Adenovirus

<400>  3

Met Arg Arg Ala His His Arg Arg Arg Arg Ala Ser His Arg Arg Met
1               5                   10                  15


Arg Gly Gly



<210>  4
<211>  7
<212>  PRT
<213>  Simian virus 40

<400>  4

Pro Lys Lys Lys Arg Lys Val
1               5